# EUROPÄISCHE PATENTSCHRIFT

(11) **EP 1 129 107 B1**
(45) Veröffentlichungstag und Bekanntmachung des Hinweises auf die Patenterteilung: **17.12.2003**
(21) Anmeldenummer: 98952496.2
(22) Anmeldetag: 19.11.1998
(51) Int. Cl.: C07K 5/08

(54) **VERFAHREN ZUR HERSTELLUNG VON L-PROLYL-L-M-SARCOLYSYL-L-P-FLUORPHENYLALANIN UND VON DERIVATEN DAVON**
METHOD FOR PRODUCING L-PROLYL-L-M-SARCOLYSYL-L-P-FLUOROPHENYLALANINE AND DERIVATIVES THEREOF
PROCEDE DE PRODUCTION DE L-PROLYL-L-M-SARCOLYSYL-L-P-FLUOROPHENYLALANINE ET DE SES DERIVES

(43) Veröffentlichungstag der Anmeldung: 05.09.2001
(73) Patentinhaber: PTC Pharma AG, 3000 Bern 25 (CH)
(72) Erfinder: MEHLEM, Francesco, CH-3048 Worblaufen (CH); DI VITTORIO, Pietro, I-20100 Milano (IT)
(74) Vertreter: Isler & Pedrazzini AG
(86) Internationale Anmeldenummer: CH9800498
(87) Internationale Veröffentlichungsnummer: WO00031119

(56) Entgegenhaltungen:
- WO-A-99/02177
- BE-A- 775 775
- US-A- 3 814 746

## Beschreibung

Die vorliegende Erfindung bezieht sich auf die ein Verfahren zur Herstellung einer pharmazeutisch aktiven Peptidverbindung, die L-m-Sarcolysin als Aminosäurebaustein enthält. Der Wirkstoff dient insbesondere zur Chemotherapie gegen Krebsleiden, besonders gegen Melanome. Bei Verwendung einer Trägersubstanz auf Basis von Cyclodextrin wird der Wirkstoff verzögert freigesetzt, was eine genügende Bioverfügbarkeit während einer ausreichend langen Zeitdauer ermöglicht.

Ein Komplex von sechs Peptiden, die m-L-Sarcolysin enthalten, ist unter dem Warennamen "Peptichemio" (lstituto Sieroterapico Milanese S. Belfanti, Milano, lT) für die Chemotherapie gegen Krebs bekannt geworden. Es wurde gefunden, dass die Aktivität der einzelnen Peptide verschieden ist und dass besonders ein Vertreter eine sehr hohe Toxizität für Melanomzellen aufweist. Die Peptide sind eine Entwicklung, welche mit dem Produkt "Melphafan", d.h. 4-[bis(2-Chlorethyl)]-amino-L-phenylalanin begonnen hat. Es wurde gefunden, dass dieses Produkt eine zytostatische Wirkung hat und sowohl für die Myelom- als auch für die Melanomtherapie eingesetzt werden kann. Zur Weiterentwicklung des Wirkstoffes wurden Derivate des Produktes hergestellt. Daraus resultierte auch das L-m-Sarcolysin [= m-{Di-2-chlorethyl)amino}-L-phenylalanin], das weiter deriviert wurde, indem Peptide hergestellt wurden, welche die modifizierte Aminosäure als Baustein enthielten. Eine Kombination der 6 Oligopeptiden L-Seryl-L-p-fluorphenylalanyl-L-msarcolysyl-ethylester; L-Prolyl-L-m-sarcolysyl-L-p fluorphenylalanin-ethylester; L-m-Sarcolysyl-N-nitro-L-arginyl-L-norvalin-ethylester; L-p-Fluorphenylalanyl-Lm-sarcolysil-L-asparagin-ethylester; Fluorphenylalanyl-glycyl-L-m-sarcolysylnorvalin-ethylester und L-m-Sarcolysyl-L-arginyl-L-lysyl-L-m-sarcolysyl-Lhistidin-methylester bildete das aktive Prinzip der Antitumormittel "Peptichemio". Von den 6 Peptiden haben sich das L-Prolyl-L-m-sarcolysyl-L-pfluorphenylalanin (PSF) und seine Niederalkylester als besonders geeignet erwiesen.

Es wurde gefunden, dass PSF eine beträchtlich höhere Zytotoxizität im Vergleich zum Peptichemio selbst zeigte (R. Levenson, et al., Radiumhemmet, Karolinska Hospital, Stockholm SE, Eur. J. Cancer Clin. Oncol.; 23: 6, 783-788, 1987). Gemäss diesen Studien wurde gefunden, dass das Peptid L-Propyl-m-sarcolysyl-L-p-fluorphenylalanin (PSF) 35 x bzw. 28 x toxischer gegen RPMl 8322 Melanomzellen war als Melphalan bzw. m-Sarcolysin. Ähnliche Unterschiede zwischen den Wirkstoffen wurden auch für andere Melanomzellinien gefunden.

Es ist Aufgabe der vorliegenden Erfindung, ein Verfahren zur Herstellung von PSF Verfügung zu stellen, das eine wirtschaftliche und sichere Herstellung des Wirkstoffes ermöglicht.

Die Herstellung einer solchen Verbindung ist in den Druckschriften BE-A-775775 und US-A-3 814 746 beschrieben. Die beschriebene Herstellung erfolgt nach dem nachstehenden Schema 1: 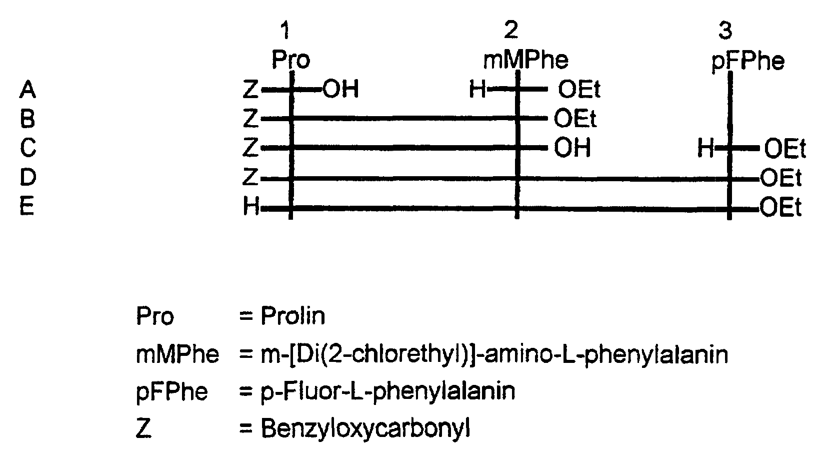 Das obige Schema zeigt in Stufe A die Kondensation des N-Carbobenzoxy-Lprolin mit dem Ethylester von m-[Di-(2-chlorethyl)-amino]-L-phenylalanin, wobei das entsprechende geschützte Peptid entsteht, wie dies bei Stufe B im Schema 1 gezeigt ist.
In Stufe C erhält man das N-Carbobenzoxy-L-prolyl-m-[di-(2-chlorethyl)-amino]-L-phenylalanin aus dem N-Carbobenzoxy-L-prolyl-m-[di-(2-chlorethyl)-amino]-L-phenylalaninethylester und anschliessend führt man die Kondensation dieser Verbindung mit p-Fluor-L-phenylalaninethylester durch, wobei man in Stufe D des Schemas 1 den Carbobenzoxy-L-prolyl-m-[di-(2-chlorethyl)amino]-Lphenylalanin-p-chlor-L-phenylalaninethylester erhält.

Man eliminiert darauf die Schutzgruppe, wobei man in Stufe E des Schemas 1 ankommt, wobei das Endprodukt der L-Prolyl-m-[di-(2-chlorethyl)amino]-Lalanin-p-fluor-L-phenylalaninethylester ist.

Die Reaktionsbedingungen sind solche, welche im allgemeinen bei Peptidsynthesen verwendet werden. Beim obigen Verfahren wird das Endprodukt mit einer Ausbeute von 30 % erhalten, bezogen auf das Ausgangsprodukt m-[Di-(2-chlorethyl)amino]-L-phenylalaninethylester, wobei die Reinigung mindestens eines Zwischenproduktes durch Säulenchromatographie auf Kieselgel durchgeführt werden muss.

Tatsächlich ist das Verfahren industriell anwendbar, jedoch ist es verhältnismässig kompliziert und führt zu einer eher ungenügenden Ausbeute.

Zieht man die Eigenschaften des Endproduktes PSF-Hydrochlorid in Betracht, ist die Verwirklichung eines anderen Herstellungsverfahrens, welches leicht industriell angewandt werden kann, welches bessere Ausbeuten gegenüber demjenigen des Standes der Technik ergibt, eine aussergewöhnlich wichtige und interessante Aufgabe der vorliegenden Erfindung.

Es wurde gefunden, dass das erfindungsgemässe Herstellungsverfahren von PSF, das eine andere Reaktionsfolge verwendet, dem Verfahren des Standes der Technik überlegen ist.

Gegenstand der vorliegenden Erfindung ist demzufolge das im Patentanspruch 1 definierte Verfahren zur Herstellung von L-Prolyl-L-msarcolysyl-L-p-fluorphenylalanin und von Estern und/oder Salzen davon.

Das erfindungsgemässe Verfahren erfolgt gemäss dem nachstehenden Schema 2: 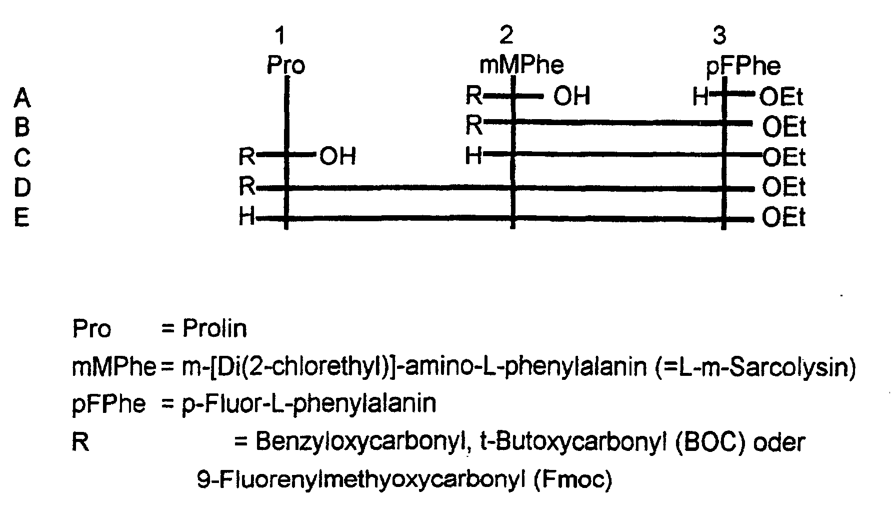 Das Verfahren umfasst folgende Verfahrensschritte, die im obigen Schema 2 dargestellt sind:
a) Kondensation von R-m-[Di-(2-chlorethyl)amino]-L-phenylalanin mit p-Fluor-L-phenylalaninethylester, wobei R-m-[Di-2-chlorethyl)amino]-Lphenylalanyl-p-ftuor-L-phenylalaninethylester erhalten wird;
b) Abspaltung der Schutzgruppe R;
c) Kondensation des im Schritt b) erhaltenen Produktes mit R-L-Prolin, wobei R-L-Prolyl-m-[di-(2-chlorethyl)amino]-L-phenylalanyl-p-fluor-Lphenylalaninethylester erhalten wird;
d) Abspaltung der Schutzgruppe R und Synthese des Hydrochlorides; R kann Benzyloxycarbonyl, t-Butyloxicarbonyl oder 9-Fluorenylmethoxycabonyl sein. R ist vorzugsweise eine Benzyloxycarbonylgruppe.

Das Verfahren gemäss der vorliegenden Erfindung zeigt eine Ausbeute von insgesamt 50 % bezogen auf das Ausgangsprodukt R-m-[Di-(2-chlorethyl)amino]-L-phenylalanin.

Das Verfahren gemäss der vorliegenden Erfindung zeigt einen grossen Vorteil zur Durchführung der Synthese des Endproduktes, da kristalline Zwischenprodukte erhalten werden, welche aussergewöhnlich leicht durch Kristallisation gereinigt werden können.

Die Merkmale und die Vorteile des erfindungsgemässen Verfahrens sollen zum besseren Verständnis durch die nachstehende Beschreibung erläutert werden. Das Tripeptid, welches nach dem erfindungsgemässen Verfahren hergestellt wird, wird gemäss dem obigen Schema 2 hergestellt. Darin ist R eine Benzyloxicarbonyl oder t-Butoxycarbonylgruppe (BOC) oder eine 9-Fluorenylmethoxycarbonylgruppe (Fmoc).

Das Verfahren sieht, wie aus dem Schema 2 hervorgeht, in Stufe A die Kondensation von R-m-[Di-2-chlorethyl)amino]-L-phenylalanin mit dem Ethylester des p-fluorphenylalanins vor, wobei in Stufe B das entsprechende geschützte Tripeptid entsteht.

In Stufe C wird die Benzyloxycarbonylgruppe entfernt, und durch eine Kondensation des R-L-Prolins mit m-[Di-(2-chlorethyl)amino]-Lphenylalanyl-p-fluor-L-phenylalaninethylester wird in Stufe D des Schemas 2 das R-L-Prolyl-m-[Di-2-chlorethyl)amino]-L-phenylalanyl-p-fluor-Lphenylalaninethylester erhalten.

Die Schutzgruppe R wird in Stufe E des Schemas 2 abgespalten, wobei das Endprodukt L-prolyl-m-[Di-(2-chlorethyl)amino]-phenylalanyl-p-fluorphenylalaninethylester ist.

Die Reaktionsbedingungen sind solche, wie sie im allgemeinen bei der Peptidsynthese üblich sind.

Das Peptid L-Prolyl-m-sarcolysyl-L-p-fluorphenylalanin (PSF) wird vorzugsweise in Form von Hydrochloriden oder Hydrobromiden hergestellt.

Das nachstende Beispiel dient der Erläuterung der vorliegenden Erfindung.

### Beispiel:

### Synthese von L-prolyl-L-m-sarcolysyl-L-p-fluorphenylalanin-ethylesterhydrochlorid

### a) N-Carbobenzoxy-L-m-sarcolysyl-L-p-fluorphenylalanin-ethylester

52,5 g L-p-Fluorphenylalaninethylester Hydrochlorid werden mit 75 ml Na2C03 (Natriumcarbonat) gesättigte Lösung und 150 ml CHCl₃ behandelt. Die Mischung wird ausgeschüttelt und die organische Phase wird getrennt und aufbewahrt. Die wässrige Phase wird mit 75 ml CHCl₃ ein zweites Mal ausgeschüttelt. Die vereinigten Chloroformextrakte werden gemischt und einmal mit Wasser gewaschen, und dann von der wässrigen Phase getrennt und auf wasserfreiem Na₂SO₄ getrocknet. Die Konzentration von Aminosäureester wird durch eine Titration mit HClO₄ (Perchlorsäure) bestimmt. Die Ausbeute entspricht ungefähr dem theoretischen Wert; sie liegt bei 98%.

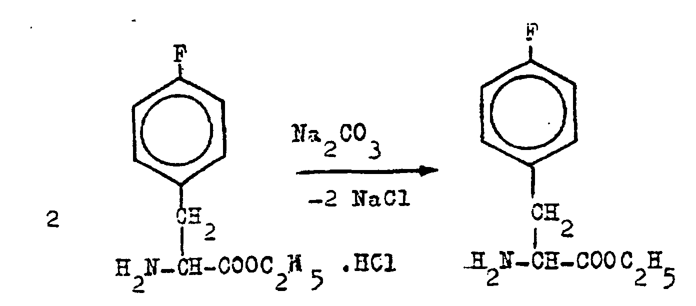

286,5 ml einer Chloroformlösung, die 0,1905 Mol L-p-Fluorphenylalaninethylester enthält, werden mit 83,7 g (0,1905 Mole) N-Cbzo-L-m-sarcolysin versetzt. Die Lösung wird auf einem Eisbad gekühlt.

Der gekühlten Lösung werden unter Rühren 41,25 g (0,200 Mol Dicyclohexylcarbodiimid - DCC) und 60 ml Chloroform dazugegeben, wobei die Lösung während 30 min. unter gleichzeitiger Kühlung ständig gerührt wird. Unter Umständen kann die Mischung zu fester Masse erstarren. ln diesem Fall wird die Masse durch Zugabe von 150 ml Chloroform wieder flüssig gemacht, wobei sie unter leichtem Erwärmen gerührt wird. Auf diese Weise wird die Auflösung des ausgefallenen Produktes beschleunigt. Die Reaktion ist 2 h nach Zugabe des DDC beendet. Das Reaktionsende wird durch TLC-Kontrolle festgestellt (Dünnschochtchromatographie; Kieselgel G-Schicht, Lösungsmittel: Chloroform + Aceton 9:1, Sichtbarmachung durch Besprühen mit verdünnter, saurer KMnO₄-Lösung). Der ausgefallene Dicyclohexylharnstoff wird durch Filtration abgetrennt. Die Lösung wird zuerst mit wenig Wasser, dann mit gesättigter Na₂CO₃-Lösung gewaschen. Die Chloroformlösung wird noch einmal mit Wasser ausgeschüttelt und dann mit Na₂SO₄ getrocknet. Das Lösungsmittel wird unter Vakuum verdampft und entfernt. Nach Trocknung werden 140,25 g leicht gelblich gefärbtes Produkt erhalten (Ausbeute 98,3%). Die gewonnene Substanz hat einen Schmelzpunkt von 123-124,5°C und ist chromatographisch homogen. Durch Kristallisation von 4,5 g Substanz aus 37,5 ml Ethylalkohol werden 3,75 g helleres Produkt gewonnen mit einem Schmelzpunkt von 125-126 °C. α_{D}²⁰: 27.7 (c = 2, CHCl₃). 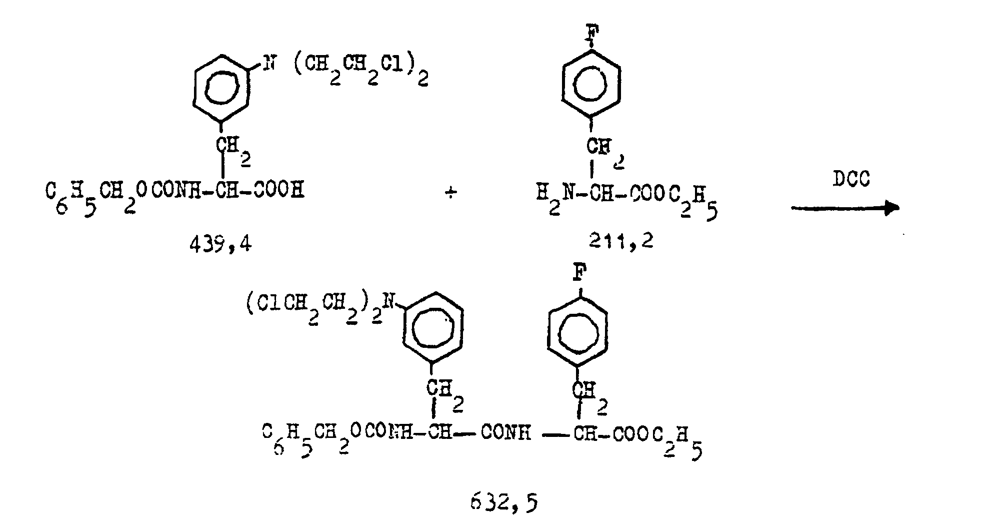 Analyse für C₃₂H₃₆Cl₂FN₃O₅
N% = 6,67 (berechnet 6,66
Cl% = 11,5 (berechnet = 11,2)

### b) L-m-Sarcolysyl-L-p-fluorphenylalanin-ethylester

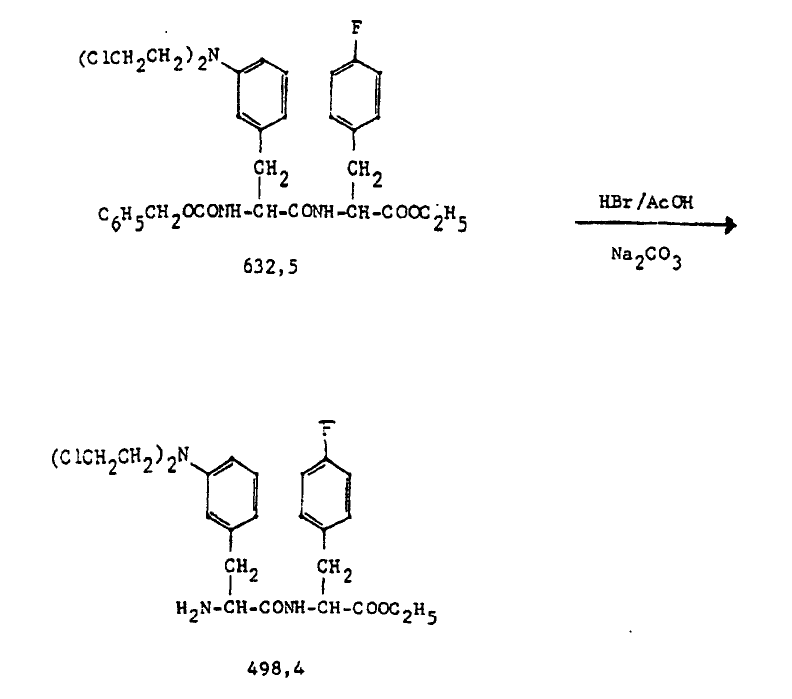

Unter Ausschluss der Luftfeuchtigkeit werden zu 390 g (0, 616 mol) die M-Carbobenzoxy-L-m-sarcolysyl-L-p-fluorphenylalanin-ethylester unter langsamem Rühren 600 ml HBr in Eisessig (33 %) zugegeben. Die Auflösung und das Aufhören der CO₂-Entwicklung findet nach 40 Minuten statt. Es wird während weiteren 20 Minuten unter Rühren stehengelassen und mit ca. 400 ml Ether verdünnt. Man giesst das gesamte in 5 l Ether, welcher unter ständigem Rühren gehalten wird, dekantiert und wäscht das ausgefallene Oel 2 x mit 2 l Ether unter Dekantieren. Das Oel wird unter Rühren mit 4 l Wasser behandelt und man erhält einen Feststoff, welcher nach ca. 30 min. durch Filtration gesammelt wird und vollständig mit insgesamt 1500 ml Wasser und 500 ml Ether gewaschen wird. Das so erhaltene Bromhydrat wird in 2 l Ethylacetat suspendiert und unter Rühren mit 450 ml gesättigter Natriumcarbonatlösung behandelt, derart bis die Lösung alkalisch ist. Nachdem die Auflösung stattgefunden hat, filtriert man auf der Nutsche, um den supendierten Dicylohexylhamstoff (sehr wenig) zu entfernen. In einem Scheidetrichter trennt man die organische Schicht von der wässerigen Phase ab, und die wässrige Phase wird mit weiteren 500 ml Ethylacetat extrahiert. Die gereinigten Extrakte werden mit 300 ml Wasser gewaschen, Na₂CO₄ getrocknet und mit Norit behandelt. Es wird filtriert und das Filtrat wird unter dem Vakuum getrocknet (40°C). Der Rückstand wird noch vor seiner Festigung in 500 bis 1000 ml Ether aufgenommen. Aus der erhaltenen Lösung wird während der Nacht ein weisses Produkt ausgefällt. Ausbeute: 247 g (80,4 %) Smp. 100 -102 °C.
α _{D}²⁰ = -7,5° (c=2, Chloroform)
TLC (BuOH/AcOH/H₂O 65:15:25; KMnO₄ verdünnt):
Eine Bande, Rf = 0,74
Analyse für C₂₄H₃₀Cl₂FN₃O₃
N% = 8,34 (berechnet 8,43)
Cl% = 14,1 (berechnet 14,2)

### c) N-Carbobenzoxy-L-prolyl-L-m-sarcolysyl-L-p-fluorphenylalaninethylester

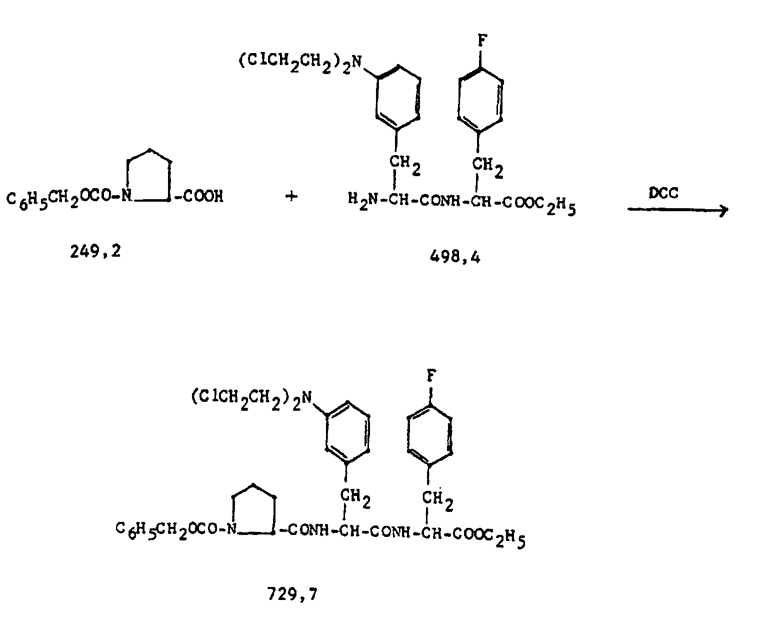

Eine Mischung von 249 g (0,5 mol) L-m-sarcolysyl-L-pfluorphenylalaninethylester, 125 g (0,5 mol) N-Cbzo-L-Prolin und 109 g (0,525 mol) DCC in 3000 ml Chloroform wird während 30 Minuten unter Rühren stehen gelassen, mit externer Kühlung während weiteren 90 Minuten bei Zimmertemperatur (TLC, Silikagel G, Chf/Me₂CO 9:1; oder mit BuOH/AcOH/H₂O 65:15:25; KMnO₄, verdünnt, sauer). Nach der Entfernung des Dicyclohexylharnstoff durch Filtration wird das Lösungsmittel unter Vakuum abgedampft und der Rückstand wird noch in flüssigem Zustand in 800 ml Ether gegossen. Von der erhaltenen Lösung fällt langsam das Produkt aus, welches auf einem Filter gesammelt wird. Ausbeute 290 g (78,5%). Smp. = 148-150°C, α_{D}²⁰= -42,4° (c=2; Chloroform)
Analyse für C₃₇H₄₃FCl₂N₄O₆
N% = 7,78% (berechnet 7,68)
Cl% = 9,6 (berechnet 9,7)

### d) L-Prolyl-L-m-sarcolysyl-L-p-fluorphenylalanin-ethylester-hydrochlorid

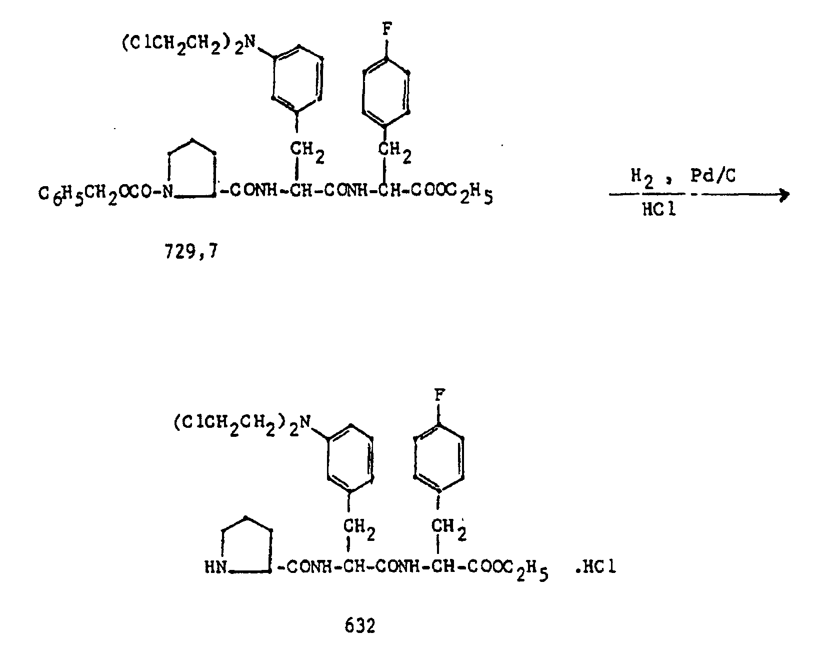

Eine Mischung von 157,5 (0,261 mol) N-Carbobenzoxy-L-prolyl-L-msarcolysyl-L-p-fluorphenylalanin-ethylester und 30 g Palladium auf Kohlenstoff 5% wird suspendiert unter einem Stickstoffstrom in 15 ml Eisessig und 1750 ml Methanol. Die Reaktionsmischung wird unter Rühren gehalten und wird unter einem Wasserstoffstrom reduziert. Nach der Beendigung der CO₂-Entwicklung (nach 4 -5 Stunden) wird eine TLC-Chromatographiekontrolle durchgeführt (Kieselgel G), wobei mit Chloroform-Aceton 9:1 eluiert wird und mit verdünntem KMnO₄ sichtbar gemacht wird.

Nachdem der Entfernung des Katalysators durch Filtration wird das Filtrat mit konzentrierter ethanolischer HCl in stöchiometrischer Menge oder wenig mehr angesäuert. Der weisse, kristalline Niederschlag, welcher sich langsam bildet, wird auf einem Filter gesammelt und mit Ethanol oder mit Ether gewaschen: 85 g. Das Filtrat wird praktisch bis zur Trockenheit konzentriert und der Rückstand wird aus Ethanol umkristallisiert: 25 g. Vollständige Ausbeute: 110 g (80, 5%); Smp. 122 - 124 °C (Änderung des Aggregatszustandes)
α_{D}²⁰ =-13,0° ± 0,5 (c= 2; MeOH)
TLC (Kieselgel G; BuOH/AcOH/H₂O 65:15:25; KMnO₄ verdünnt: eine
Bande Rf = 0,54.
Analyse für C₂₉H₃₈Cl₃FN₄O4
N % = 8,93% (berechnet 8,86)
Cl % = 16,7 % (berechnet 16,8)
Cl-% = 5,65% (berechnet 5,6)

## Patentansprüche

1. Verfahren zur Herstellung von L-Prolyl-L-m-sarcolysyl-L-pfluorphenylalanin, eines Niederalkylesters und/oder von Säureadditionssalzen davon **dadurch gekennzeichnet, dass** L-p-Fluorphenylalanin mit einer geschützten Carboxyl-Gruppe mit L-m-Sarcolysin mit einer geschützten Aminogruppe und einer aktivierten Carboxy-Gruppe umgesetzt wird, wobei L-m-Sarcolysyl-L-p-fluorphenylalanin mit einer geschützten Aminogruppe und einer geschützten Carboxy-Gruppe erhalten wird und anschliessend die Aminoschutzgruppe abgespalten wird; danach das erhaltene L-m-Sarcolysyl-L-p-fluorphenylalanin mit einer geschützten Carboxy-Gruppe mit Prolin mit einer geschützten Aminogruppe und einer aktivierten Carboxy-Gruppe umgesetzt wird, wobei L-Prolyl-L-m-sarcolysyl-L-pfluorphenylalanin mit einer geschützen Aminogruppe erhalten wird und die Amino-Schutzgruppe abgespalten und gegebenenfalls die Niederalkylestergruppe abgespalten oder in eine andere Estergruppe übergeführt wird und/oder die erhaltene Verbindung in ein Säureadditionssalz übergeführt wird.

2. Verfahren nach Anspruch 1, **dadurch gekennzeichnet dass** die Kondensation unter Kühlung in einem wasserfreien Medium durchgeführt wird, z.B. in Chloroform.

3. Verfahren nach Anspruch 1 oder 2, **dadurch gekennzeichnet, dass** die aktivierten Carboxy-Gruppen durch Behandlung mit Dicyclohexylcarbodiimid aktiviert wurden.

4. Verfahren nach einem der Ansprüche 1 bis 3, **dadurch gekennzeichnet, dass** die Carboxy-Schutzgruppe von L-p-Fluorphenylalanin eine Niederalkylestergruppe, vorzugsweise eine Ethyestergruppe ist.

5. Verfahren nach einem der Ansprüche 1 bis 4, **dadurch gekennzeichnet, dass** die Aminoschutzgruppe des L-m-Sarcolysin eine Carbobenzoxy-Gruppe ist.

6. Verfahren nach einem der Ansprüche 1 bis 5, **dadurch gekennzeichnet, dass** die Abspaltung der Aminoschutzgruppe des L-m-Sarcolysyl-L-p fluorphenylalanins mit einer geschützten Aminogruppe durch Behandlung mit Bromwasserstoff in Eisessig durchgeführt wird.

7. Verfahren nach einem der Ansprüche 1 bis 6, **dadurch gekennzeichnet, dass** die Abspaltung der Aminoschutzgruppe des L-Prolyl-L-m-sarcolysyl-L-p-fluorphenylalanins mit einer geschützten Aminogruppe durch Reduktion mit Wasserstoff in Gegenwart von Palladium auf Kohlenstoff durch geführt wird.

## Claims

1. Method for the production of L-prolyl-L-m-sarcolysyl-L-p-fluorophenylalanine, of a lower alkyl ester and/or of acid addition salts thereof, **characterized in that** L-p-fluorophenylalanine with a protected carboxyl group is reacted with L-m-sarcolysine with a protected amino group and an activated carboxyl group, wherein L-m-sarcolysyl-L-p-fluorophenylalanine is obtained with a protected amino group and with a protected carboxyl group and the amino protective group is then split off, the resulting L-m-sarcolysyl-L-p-fluorophenylalanine with a protected carboxyl group is then reacted with proline presenting a protected amino group and an activated carboxyl group, wherein L-prolyl-L-m-sarcolysyl-L-p-fluorophenylalanine is obtained with a protected amino group and that the amino protective group is split off and possibly the lower alkyl ester group is split off or is transformed in another ester group and/or the resulting compound is transformed into an acid addition salt.

2. Method according to claim 1, **characterized in that** the condensation is performed during cooling in an anhydrous medium, e.g. chloroform.

3. Method according to claim 1 or 2, **characterized in that** the activated carboxyl groups have been activated through treatment with dicyclohexylcarbodiimide.

4. Method according to one of claims 1 to 3, **characterized in that** the carboxyl protective group of L-p-fluorophenylalanine is a lower alkyl ester group, preferably an ethyl ester group.

5. Method according to one of claims 1 to 4, **characterized in that** the amino protective group of L-m-sarcolysine is a carbobenzoxyl group.

6. Method according to one of claims 1 to 5, **characterized in that** the split off of the amino protective group of L-m-sarcolysyl-L-p-fluorophenylalanine with a protected amino group is conducted with the treatment with hydrogen bromide in glacial acetic acid.

7. Method according to one of claims 1 to 6, **characterized in that** the split off of the amino protective group of L-prolyl-L-m-sarcolysyl-L-p-fluorophenylalanine with a protected amino group is performed through reduction by hydrogen in presence of palladium on carbon.

## Revendications

1. Procédé de production de L-prolyl-L-m-sarcolysyl-L-p-fluorophénylalanine, d'un ester d'alkyle inférieur de celui-ci et/ou d'un sel d'addition d'acides de celui-ci, **caractérisé en ce que** L-p-fluorophenylalanine avec un groupe carboxy-protecteur est transformé avec L-m-sarcolysine avec un groupe amino-protecteur et un group carboxyle activé, où L-m-sarcolysyl-L-p-fluorophenylalanine est obtenu avec un groupe amino-protecteur et avec un groupe carboxy-protecteur et où le groupe amino-protecteur est séparé ensuite, où le L-m-sarcolysyl-L-p-fluorophenylalanine avec un groupe carboxy-protecteur qui en résulte est ensuite transformé avec de la proline présentant un group amino-protecteur et un group carboxyle activé, où L-prolyl-L-m-sarcolysyl-L-p-fluorophenylalanine est obtenu avec un groupe amino-protecteur et **en ce que** le group amino-protecteur est séparé et éventuellement le groupe d'alkyl inférieur est séparé ou est transformé en un autre groupe d'ester et/ou le composé qui en résulte est transformé en un sel d'addition d'acides.

2. Procédé selon la revendication 1, **caractérisé en ce que** la condensation est effectué sous refroidissement dans une composition anhydre, par exemple dans du chloroforme.

3. Procédé selon la revendication 1 ou 2, **caractérisé en ce que** les groupes carboxyles activés ont été activé par traitement avec du dicyclohexylcarbodiimide.

4. Procédé selon l'une des revendications 1 à 3, **caractérisé en ce que** le groupe carboxy-protecteur du L-p-fluorophenylalanine est un groupe d'ester d'alkyl inférieur, préférablement un group d'éthyle ester.

5. Procédé selon l'une des revendications 1 à 4, **caractérisé en ce que** le group amino-protecteur du L-m-sarcolysine est un group carbobenzoxyle.

6. Procédé selon l'une des revendications 1 à 5, **caractérisé en ce que** la séparation du group amino-protecteur du L-m-sarcolysyl-L-p-fluorophenylalanine avec un groupe amino-protecteur est effectué avec un traitement avec du bromure d'hydrogène dans de l'acide acétique glacial.

7. Procédé selon l'une des revendications 1 à 6, **caractérisé en ce que** la séparation du group amino-protecteur du L-prolyl-L-m-sarcolysyl-L-p-fluorophenylalanine avec un groupe amino-protecteur est effectué par la réduction par hydrogène en présence du palladium sur du carbone.
